(19) Europäisches Patentamt
European Patent Office
Office européen des brevets

(11) **EP 4 729 042 A1**

(12) **EUROPEAN PATENT APPLICATION**

(43) Date of publication:
**22.04.2026 Bulletin 2026/17**

(21) Application number: **24206569.6**

(22) Date of filing: **15.10.2024**

(51) International Patent Classification (IPC):
**A61K 6/17** *(2020.01)* **A61K 6/77** *(2020.01)*
**A61K 6/887** *(2020.01)*

(52) Cooperative Patent Classification (CPC):
(C-Sets available)
**A61K 6/887; A61K 6/17; A61K 6/77** (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Stick Tech OY
20521 Turku (FI)**

(72) Inventors:
• **LASSILA, Lippo
21630 Lielax (FI)**
• **GAROUSHI, Sufyan
20660 Littoinen (FI)**
• **VALLITTU, Pekka
21620 Kuusisto (FI)**
• **SÄILYNOJA, Eija
20660 Littoinen (FI)**

(74) Representative: **Laine IP Oy
Porkkalankatu 24
00180 Helsinki (FI)**

(54) **A DENTAL RESTORATIVE COMPOSITION**

(57) The present invention relates to a dental restorative composition, comprising 10-30 wt-% of a resin and 45-80 wt-% of silanated inert glass particles having an average particle size of 0.2-0.9 μm.

EP 4 729 042 A1

(52) Cooperative Patent Classification (CPC): (Cont.)

C-Sets
**A61K 6/887, C08L 33/08;**
**A61K 6/887, C08L 33/10**

**Description**

FIELD

The present invention relates to dental restorative compositions.

BACKGROUND AND OBJECTS

Tooth decay, also known as dental caries or cavities, is the breakdown of teeth due to acids made by bacteria. Caries is treated by removing the carious lesions and replacing them with restorative material. Presently the restorative material is mainly based on polymers.

A disadvantage of some dental restorative compositions is that they are visible to the naked eye, i.e. one can see the limit between the tooth and the restoration.

The chameleon effect is an aesthetic property that enables the restorative material to match the colour of its surroundings. Composite materials that exhibit this property eliminate aesthetic faults dramatically, and approximatively 90 % of such restorations can be successfully achieved using a single shade of the material. Some dental restorative compositions use the so-called chameleon effect successfully, but may lack in mechanical properties.

An aim of the present invention is thus to develop a dental restorative composition that benefits from the chameleon effect, yet have good mechanical properties.

SUMMARY OF THE INVENTION

The invention is defined by the features of the independent claim. Some specific embodiments are defined in the dependent claims.

According to an aspect, there is provided a dental restorative composition, comprising 10-30 wt-% of a resin and 45-80 wt-% of silanated inert glass particles having an average particle size of 0.2-0.9 $\mu$m.

BRIEF DESCRIPTION OF THE DRAWINGS

Figure 1 illustrates some translucency parameters of some materials according to an embodiment as well as of some comparative examples.

Figure 2 illustrates goniophotometer measurements for some samples.

Figure 3 shows a first caries made in a first dental model.

Figures 4 and 5 show the caries of Figure 3 restorated with a material according to an embodiment as well as some commercial products.

Figure 6 shows a second caries in a second dental model.

Figures 7 and 8 show the caries of Figure 6 restorated with a material according to an embodiment as well as some commercial products.

DETAILED DESCRIPTION

In the context of the present application, an average length of fibres is determined as follows. The fibres are photographed with a stereo-microscope at a magnification of 6.5 x (or alternatively a scanning electron microscope). The photos are then processed with Image-J processing program to determine the lengths of the fibres. The total number of fibres taken into the calculation is 500. Thereafter, the fibres are divided into a number of fractions with 0.1 mm intervals according to their length. The fibre lengths in each 0.1 mm interval are added together. The average fibre length is taken to be value where the lengths of the shorter fibres and longer fibres are deemed to be the same. This measurement method is described in Lassila et al., "Mechanical properties of fiber reinforced restorative composite with two distinguished fiber length distribution", Journal of the mechanical behavior of biomedical materials 60 (2016) 331-338, section 2.5 on page 333. A diameter of the fibres (i.e. the diameter of a cross-section), if not provided by the manufacturer, can also be determined in this way.

The same determination method can be used for determining an average size of particles (with 1000 x magnification instead of 6.5 x), and the measured dimension is the largest dimension of the particle. Most typically, the average particle size of especially the particles is provided by the manufacturer of the particles. For both the fibres and particles, the average is the arithmetic average.

Weight percentages (wt-%) refer to the weights in the total composition or in a mixture of resins, as can be deduced from the context. In the claims, the wt-% are of the total weight of the composition.

In this specification, particles are different from fibres, and fibres have a length that is at least 5 x the diameter of the fibre.

According to an aspect of the present invention, there is provided a dental restorative composition, comprising 10-30 wt-% of a resin and 45-80 wt-% of silanated inert glass particles having an average particle size of 0.2-0.9 $\mu$m.

As shown below in the Experimental part, such dental restorative composition exhibits the chameleon effect, in addition to good mechanical properties. Indeed, the present dental restorative composite showed a dentin-like light scattering effect while the commercial products had an enamel-like light scattering effect. The advantage of dentin-like scattering effect is that the restoration can be practically invisible to the naked eye, as it mimics the colour of its surroundings. This has the advantage that a dentist needs a smaller amount of different shades of dental restorative composites. The mechanical properties were good and in any case sufficient for dental restorative composites, as for example the fracture toughness was close to that of dentin.

The amount of resin (which may be also a mixture of resins) is 10-30 wt-% of the weight of the total composition. According to an embodiment, the amount of resin is 15-25 wt-%. The amount of resin can be for example from 10, 12, 14, 15, 17, 18, 20, 22, 24, 25, 26 or 28 wt-% up to 12, 14, 15, 17, 18, 20, 22, 24, 25, 26, 28 or 30 wt-%.

The composition also comprises 45-80 wt-% of silanated inert glass particles, of the total weight of the composition. The amount of silanated inert glass particles can be for example from 45, 47, 50, 53, 55, 57, 60, 63, 65, 67, 70, 73, 75 or 77 wt-% up to 47, 50, 53, 55, 57, 60, 63, 65, 67, 70, 73, 75, 77 or 80 wt-%.

According to an embodiment, the amount of the silanated inert glass particles is 45-60 wt-%, and the composition further comprises 20-30 wt-% of inert glass fibres, the glass fibres having a diameter of 4-8 $\mu$m and a distribution of length of 50-200 $\mu$m.

Thus, when fibres are also used, the amount of particles is lower than if they are used alone. According to an embodiment, the amount of glass fibres is 24-28 wt-%.

When glass fibres are used, they have a diameter of 4-8 $\mu$m, for example from 4, 5, 6 or 7 $\mu$m up to 5, 6, 7 or 8 $\mu$m. The distribution of length of the fibres is 50-200 $\mu$m. Indeed, the distribution of length of the fibres can be from 50, 70, 75, 80, 100, 120, 135, 150, 170 or 190 $\mu$m up to 70, 75, 80, 100, 120, 135, 150, 170, 190 or 200 $\mu$m.

The fibres are typically randomly oriented in the finished product. Using short randomly oriented micro-scaled fibres improves the mechanical stability and ability of the adhesive layer at the interface to arrest crack propagation.

The degree of silanation of the inert glass particles may be 1-15 wt-%. The degree of silanation may be for example from 1, 2, 3, 4, 5, 6, 7, 8, 9, 10, 11, 12 or 13 wt-% up to 2, 3, 4, 5, 6, 7, 8, 9, 10, 11, 12, 13, 14 or 15 wt-%. For example, a degree of silanation of 6 wt-% means that the particles have been immersed to a solution containing 6 wt-% of silane molecules, of the total weight of the particles. The fibres may also be silanated, using the same degree of silanation as the glass particles, but selected independently from the degree of silanation of the glass particles. It is believed that the process of immersing the particles and/or fibres into the silane solution results in essentially all of the surface of the particles and/or fibres to be covered by the silane solution, and after drying, of the silane, while taking into account the amount of silane molecules used.

The silanated inert glass particles have an average particle size of 0.2-0.9 $\mu$m. According to an embodiment, the average particle size of the silanated inert glass particles is 0.4-0.7 $\mu$m. The average particle size (measured as indicated above) can be from 0.2, 0.25, 0.3, 0.35, 0.4, 0.45, 0.5, 0.55, 0.6, 0.65, 0.7, 0.75, 0.8 or 0.85 $\mu$m up to 0.25, 0.3, 0.35, 0.4, 0.45, 0.5, 0.55, 0.6, 0.65, 0.7, 0.75, 0.8, 0.85 or 0.9 $\mu$m.

The inert glass used for the particles and optional fibres may be any inert glass, for example independently selected from E-glass, S-glass and mixtures thereof.

According to an embodiment, the resin is selected from a group consisting of methyl methacrylate, ethyl methacrylate, n-butyl methacrylate, isobutyl methacrylate, 2-ethylhexyl methacrylate, cyclohexyl methacrylate, isobornyl methacrylate, tetrahydrofurfuryl methacrylate, benzyl methacrylate, morpholinoethyl methacrylate, diethylene glycol dimethacrylate, triethylene glycol dimethacrylate, diurethane dimethacrylate, 2,2-bis(4-(2-hydroxy-3-methacryloxy)phenyl)propane, acrylic acid, epoxy, bisphenol A-glycidyl methacrylate (bis-GMA), bisphenol A ethoxylate dimethacrylate (bis-EMA), urethane dimethacrylate (UDMA), trimethylolpropane ethoxylate triacrylate and mixtures thereof.

The dental composition may comprise very small amounts of active components, such as bioactive or partially reactive glass ionomer fillers containing elements such as oxides of silicon (Si), calcium (Ca), phosphorus (P), barium (Ba), magnesium (Mg), potassium (K), titanium (Ti), fluorine (F), strontium (Sr), zinc (Zn), cerium (Ce), niobium (Nb) or other compounds of said elements, colour pigments, inert ceramics, inert silica, hydroxyl apatite (HA) or other Ca-phosphates, $Al_2O_3$, $ZrO_2$, Ag, zerogels, bioactive glasses or particles containing functional bioactive or therapeutically active molecules, antigens, antibiotics, disinfectants, radio-opaque materials, organic acids such as maleic acids, polyacrylic acid, or the like. The therapeutically active molecules may include antimicrobial molecules, or the particles may be antimicrobial themselves.

It is to be understood that the embodiments of the invention disclosed are not limited to the particular structures, process steps, or materials disclosed herein, but are extended to equivalents thereof as would be recognized by those ordinarily skilled in the relevant arts. It should also be understood that terminology employed herein is used for the purpose of describing particular embodiments only and is not intended to be limiting.

Furthermore, the described features, structures, or characteristics may be combined in any suitable manner in one or more embodiments. In the description, numerous specific details are provided, such as examples of lengths, widths, shapes, etc., to provide a thorough understanding of embodiments of the invention.

The verbs "to comprise" and "to include" are used in this document as open limitations that neither exclude nor require the existence of also un-recited features. The features recited in depending claims are mutually freely combinable unless otherwise explicitly stated. Furthermore, it is to be understood that the use of "a" or "an", i.e. a singular form, throughout this document does not exclude a plurality.

EXPERIMENTAL PART

Several different samples according to the present description were prepared, and compared to samples prepared using commercial products. Various measurements were made, as will be explained below.

The following materials were used in the Examples. When a distribution of length or an average particle size is given, it is the value indicated by the manufacturer.

- E-glass fibres having a diameter of 6 $\mu$m and a distribution of length of 50-200 $\mu$m (i.e. 90 wt-% of the fibres are within this range), GC Microfibre EFDE 90-01 from GC Corp. Japan
- filler 1: Schott UltraFine UF0.4 $\mu$m, G018-053 from Schott, Germany
- filler 2: Schott UltraFine UF0.7 $\mu$m, G018-053 from Schott, Germany
- silane, KBM-5803 from Shin-Etsu Chemical Co. Ttd. Japan
- bis-EMA (bisphenol A ethoxylate dimethacrylate), Esschem, X970 0000, lot 806-136
- bis-EMA3 (bisphenol A ethoxylate dimethacrylate), x970 0000 / P5 from Esschem, USA
- bis-EMA4 (bisphenol A ethoxylate dimethacrylate), B6286 from Tokyo Chemical Industry Co., Ltd., Japan
- TEGDMA (triethylene glycol dimethacrylate), 261548 - 1L from Sigma-Aldrich Co., USA
- UDMA (urethane dimethacrylate), X850700 from Esschem, USA
- camphorquinone (CQ), 102410085 from Sigma-Aldrich, USA was used as a photoinitiator
- ethyl 4-(dimethylamino) benzoate (EPA), E24905 -100g from Sigma-Aldrich, USA was used as a photoinitator
- Essentia Universal, 230329A from GC Corp., Japan
- G-ænial® Universal Injectable shade A2, 230124A from GC Corp., Japan
- G-aenial® A'Chord shade A2, 230202A from GC Corp., Japan
- 3M Filtek™ Universal Restorative shade A2, lot: NF30796, from 3M ESPE Dental Products, USA
- Tetric® Prime shade A2, lot. Z03R0G, from Ivoclar Vivadent AG, Liechtenstein
- Omnichroma, lot: 047E32, from Tokuyama Dental Corporation, Japan
- Venus® Diamond One, lot: M010207, from Kulzer GmbH, Germany
- Ceram.x Spectra™ ST, lot: 2206000483, Dentsply DeTrey GmbH, Germany

Silanisation of the fillers

The fillers were silanised by mixing 5 g of silane, 50 ml of a mixture of 95 vol-% of ethanol and 5 vol-% of water. The mixture was stirred for 30-60 min with a magnetic stirrer. 50 g of the unsilanised fillers were added to the solution in small portions. The decanter was covered and mixing continued for two hours at 50 °C. Thereafter, the stirring was set to a minimum and the heating left on overnight.

On the next day, the solution was vaporised from the decanter by opening the cover. When all the liquid was gone, the magnet was removed and the fillers were set in to a vacuum oven to dry. The settings were 90 °C 2-3 h in 5 mbar vacuum. After the set time, the oven was turned off, but the vacuum was left inside for overnight. On the following day, the fillers were removed from the vacuum oven, the decanter was covered with aluminium foil and the fillers were set inside a desiccator to keep them dry.

This led to a silanation of approximatively 6 wt-%.

Preparation of tooth models

Tooth models were made of four different shades of a dental composite, namely DenFil, Light-cured Hybrid Composite resin from Vericom Co., LTD, Korea. The shades were A1 (lot DF1929A1 ), A3 (lot DF2167A3), A4 (lot DF0703A4) and C3 (lot: DF3128C3). Preparation of the tooth models was carried out in the conventional manner.

**Example 1 (E1)**

The resin mixture had the composition shown in Table 1.

Table 1

| Component | wt-% |
|-----------|------|
| UDMA | 80 |
| TEGDMA | 15 |
| bis-EMA 3 | 5 |
| CQ | 0.5 |
| EPA | 0.5 |

The composition tested in Example 1 contained 15 wt-% of the resin mixture, 28 wt-% of the E-glass fibres and 57 wt-% of silanated particulate fillers 1.

**Example 2 (E2)**

The resin mixture had the composition shown in Table 2.

Table 2

| Component | wt-% |
|-----------|------|
| UDMA | 80 |
| TEGDMA | 2 |
| bis-EMA 3 | 18 |
| CQ | 0.5 |
| EPA | 0.5 |

The composition tested in Example 2 contained 14.9 wt-% of the resin mixture, 27.6 wt-% of the E-glass fibres and 57.5 wt-% of silanated particulate fillers 1.

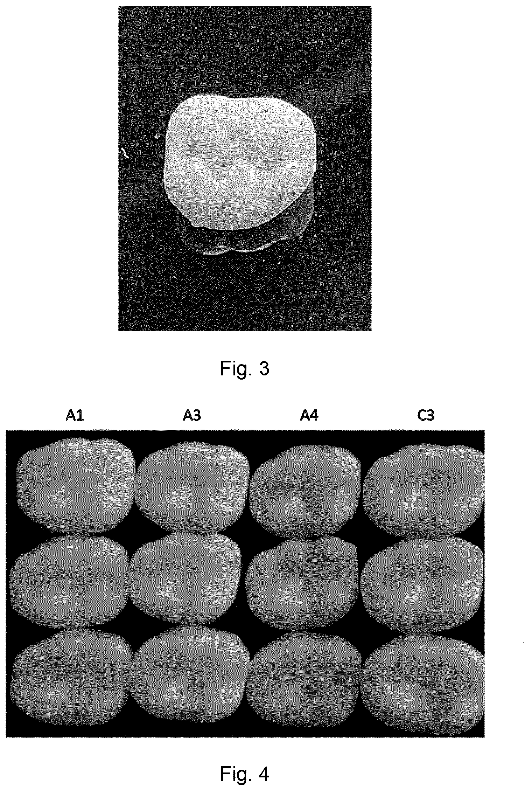

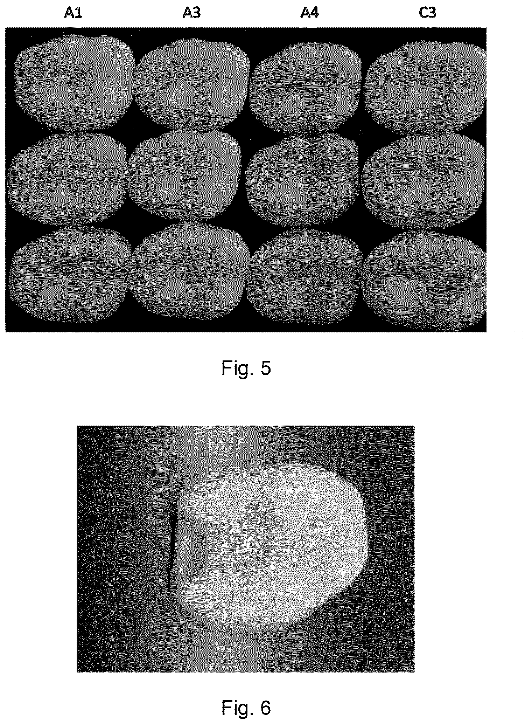

The composition was used to fill in a caries in a tooth model. An occlusal surface cavity (shown in Figure 3) was prepared on the model, and filled with the composition, applied in layers. Each layer was light cured with GC's light curing device D-Light Pro, HP-setting for 2 0s. Afterwards the surface was polished with dental polishers. Another cavity (shown in Figure 6) was prepared on another model, and filled in the same manner.

**Example 3 (E3)**

The resin mixture had the composition shown in Table 3.

Table 3

| Component | wt-% |
|-----------|------|
| UDMA | 80 |
| TEGDMA | 2 |
| bis-EMA 3 | 9 |
| bis-EMA 4 | 9 |

(continued)

| Component | wt-% |
|---|---|
| CQ | 0.5 |
| EPA | 0.5 |

The composition tested in Example 3 contained 14.9 wt-% of the resin mixture, 27.5 wt-% of the E-glass fibres and 57.5 wt-% of silanated particulate fillers 1.

**Example 4 (E4)**

The resin mixture had the composition shown in Table 4.

Table 4

| Component | wt-% |
|---|---|
| UDMA | 81 |
| TEGDMA | 2 |
| bis-EMA 3 | 8.5 |
| bis-EMA 4 | 8.5 |
| CQ | 0.5 |
| EPA | 0.5 |

The composition tested in Example 4 contained 14.9 wt-% of the resin mixture, 27.5 wt-% of the E-glass fibres and 57.5 wt-% of silanated particulate fillers 1.

**Example 5 (E)**

The resin mixture had the composition shown in Table 5.

Table 5

| Component | wt-% |
|---|---|
| UDMA | 79.1 |
| bis-EMA | 19.9 |
| CQ | 0.5 |
| EPA | 0.5 |

The composition tested in Example 5 contained 25.2 wt-% of the resin mixture, 24.9 wt-% of the E-glass fibres and 49.9 wt-% of silanated particulate fillers 2.

**Example 6 (E6)**

The resin mixture had the composition shown in Table 6.

Table 6

| Component | wt-% |
|---|---|
| UDMA | 78.8 |
| TEGDMA | 5.3 |
| bis-EMA | 15.0 |
| CQ | 0.5 |

(continued)

| Component | wt-% |
|-----------|------|
| EPA | 0.5 |

The composition tested in Example 6 contained 25.4 wt-% of the resin mixture, 24.9 wt-% of the E-glass fibres and 49.7 wt-% of silanated particulate fillers 2.

**Example 7 (E7)**

The resin mixture had the composition shown in Table 7.

Table 7

| Component | wt-% |
|-----------|------|
| UDMA | 79.2 |
| TEGDMA | 9.9 |
| bis-EMA | 9.9 |
| CQ | 0.5 |
| EPA | 0.5 |

The composition tested in Example 7 contained 25.3 wt-% of the resin mixture, 24.9 wt-% of the E-glass fibres and 49.8 wt-% of silanated particulate fillers 2.

**Example 8 (E8)**

The resin mixture had the composition shown in Table 8.

Table 8

| Component | wt-% |
|-----------|------|
| UDMA | 79.3 |
| TEGDMA | 14.8 |
| bis-EMA | 4.9 |
| CQ | 0.5 |
| EPA | 0.5 |

The composition tested in Example 8 contained 25.2 wt-% of the resin mixture, 25 wt-% of the E-glass fibres and 49.8 wt-% of silanated particulate fillers 2.

**Example 9 (E9)**

The resin mixture had the composition shown in Table 9.

Table 9

| Component | wt-% |
|-----------|------|
| UDMA | 79.3 |
| TEGDMA | 19.7 |
| CQ | 0.5 |
| EPA | 0.5 |

The composition tested in Example 9 contained 25.2 wt-% of the resin mixture, 25 wt-% of the E-glass fibres and 49.8 wt-% of silanated particulate fillers 2.

### Example 10 (E10)

The resin mixture had the composition shown in Table 10.

Table 10

| Component | wt-% |
| --- | --- |
| UDMA | 79.4 |
| bis-EMA | 19.7 |
| CQ | 0.5 |
| EPA | 0.5 |

The composition tested in Example 10 contained 25.3 wt-% of the resin mixture and 74.7 wt-% of silanated particulate fillers 2.

### Example 11 (E11)

The resin mixture had the composition shown in Table 11.

Table 11

| Component | wt-% |
| --- | --- |
| UDMA | 79.3 |
| TEGDMA | 4.9 |
| bis-EMA | 14.8 |
| CQ | 0.5 |
| EPA | 0.5 |

The composition tested in Example 11 contained 25.2 wt-% of the resin mixture and 74.8 wt-% of silanated particulate fillers 2.

### Example 12 (E)

The resin mixture had the composition shown in Table 12.

Table 12

| Component | wt-% |
| --- | --- |
| UDMA | 79.5 |
| TEGDMA | 9.8 |
| bis-EMA | 9.8 |
| CQ | 0.5 |
| EPA | 0.5 |

The composition tested in Example 12 contained 25.2 wt-% of the resin mixture and 74.8 wt-% of silanated particulate fillers 2.

**Example 13 (E13)**

The resin mixture had the composition shown in Table 13.

Table 13

| Component | wt-% |
|---|---|
| UDMA | 79.2 |
| TEGDMA | 14.8 |
| bis-EMA | 5.1 |
| CQ | 0.5 |
| EPA | 0.5 |

The composition tested in Example 13 contained 25.3 wt-% of the resin mixture and 74.7 wt-% of silanated particulate fillers 2.

**Example 14 (E14)**

The resin mixture had the composition shown in Table 14.

Table 14

| Component | wt-% |
|---|---|
| UDMA | 78.9 |
| TEGDMA | 20.1 |
| CQ | 0.5 |
| EPA | 0.5 |

The composition tested in Example 14 contained 25.2 wt-% of the resin mixture and 74.8 wt-% of silanated particulate fillers 2.

**Comparative examples**

As comparative examples, samples prepared using commercial dental compositions were used as shown in Table 15.

Table 15

| Comparative example | Composition used |
|---|---|
| C1 | Venus Diamond One (lot. N010208, Hereaus Kulzer) |
| C2 | 3M Filtek Universal |
| C3 | Tetric Prime (lot. Z04DP2, Ivolar Vivadent) |
| C4 | Omnichroma |
| C5 | Ceram X Spectra |
| C6 | G-aenial A'Chord |
| C7 | Essentia Universal |
| C8 | G-aenial Injectable |

The compositions of Comparative examples C1 and C4 were (independently) used to fill in a caries in two tooth models, as explained in Example 2.

Translucency test

Discs for the translucency tests were prepared in a mould. The discs were cylindrical with a height of 1 mm or 2 mm, and a diameter of 8 mm. Both upper and lower side of the mould was covered with a Mylar® film (thickness 100 μm) and microscope glass plate. The discs were polymerised with light using D-Light Pro from GC, HP-setting (high power), 20 s from both sides of the disc. The discs were stored in an incubator at least 48 h hours before testing. Both surfaces of each disc were polished 4000-grit SiC-paper.

The translucency measurement was performed with a colour measuring device, Spectrophotometer CM-700d with Spectra Magic NX-program. After calibrating the instrument, a disc was placed on top of the target mask by covering the measuring area. Each disc was measured against a white and a black background.

For each disc was Delta E-values (translucency parameter) by comparing the values given from the black background and from white background was calculated.

The results are given in Tables 16 and 17 below, where three different values are presented:

L*which presents the lightness - from black to white
a* which presents the colour "shade" - from green to red
b* which presents the colour "shade" - from blue to yellow

$$\Delta E_{ab}^{*} = \sqrt{(L_2^* - L_1^*)^2 + (a_2^* - a_1^*)^2 + (b_2^* - b_1^*)^2}$$

The results with number "2" are for the samples having a thickness of 2 mm, the others for the samples having a thickness of 1 mm.

A translucency parameter TP was obtained using equation (1)

$$TP = [(Lb − Lw)2 + (ab − aw)2 + (bb − bw)2]½ \qquad (1)$$

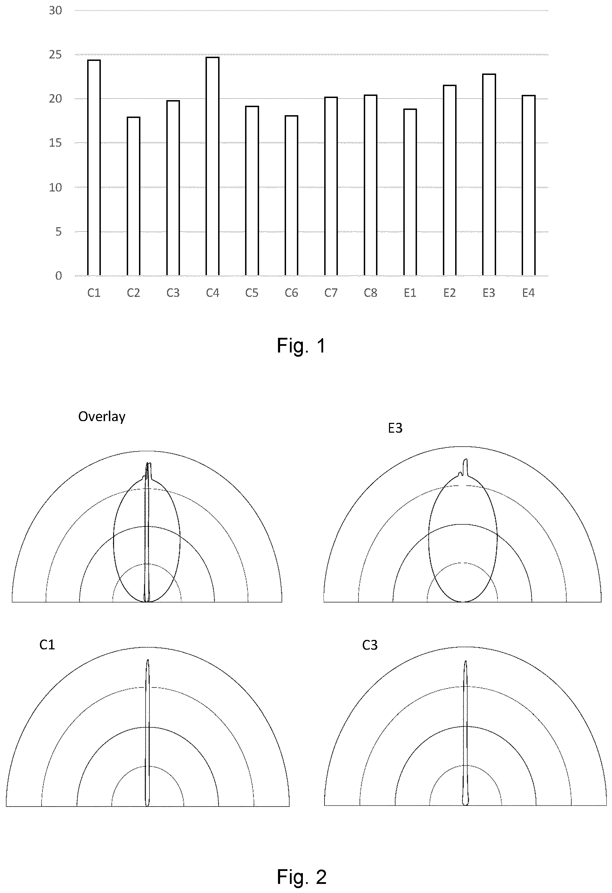

wherein b refers to white background and w to black background. The values of Lb, Lw, ab, aw, bb and bw were obtained using as colour measuring device a spectrophotometer CM-700d (Konica Minolta Sensing Inc., Japan). The values are given in Tables 16 and 17 and the translucency parameters TP shown in Table 16 also in Figure 1.

Table 16

| Example | White background | | | Black background | | | TP |
|---|---|---|---|---|---|---|---|
| | | Red | Yellow | | Red2 | Yellow2 | |
| | L*(D65) | a*(D65) | b*(D65) | L*(D65) | a*(D65) | b*(D65) | ΔE |
| E1 | 79,71 | -1,43 | 6,87 | 62,2 | -2,31 | 0,01 | 19 |
| E2 | 76,76 | -1,05 | 6,05 | 56,22 | -2,07 | -0,23 | 22 |
| E3 | 76,82 | -1,21 | 8,23 | 55,35 | -2,31 | 0,75 | 23 |
| E4 | 76,24 | -1,48 | 9,12 | 57,49 | -2,48 | 1,28 | 20 |
| C1 | 78,24 | 0,05 | 11,71 | 56,07 | -1,14 | 1,67 | 24 |
| C2 | 78,79 | 1,81 | 17,58 | 64,81 | -0,96 | 6,75 | 18 |
| C3 | 76,82 | 2,71 | 16,55 | 61,92 | -1,15 | 4,14 | 20 |
| C4 | 78,57 | -1,08 | 10,24 | 55,53 | -2,76 | 1,61 | 25 |
| C5 | 76,74 | 2,65 | 20,48 | 64,13 | -1,51 | 6,7 | 19 |
| C6 | 76,11 | 2,02 | 14,71 | 61,55 | -0,79 | 4,43 | 18 |
| C7 | 77,97 | 1,66 | 13,34 | 61,58 | -1,46 | 2,05 | 20 |
| C8 | 77,03 | 3,01 | 16,39 | 62,41 | -1,12 | 2,81 | 20 |

Table 17

| Example | White background | | | Black background | | | TP |
|---|---|---|---|---|---|---|---|
| | | Red | Yellow | | Red2 | Yellow 2 | |
| | L* | a* | b* | L* | a* | b* | ΔE |
| E5 | 81,7 | -0,9 | 10,8 | 51,5 | -1,5 | 5,1 | 30,7 |
| E6 | 80,3 | -1,2 | 11,8 | 53,8 | -1,8 | 5,0 | 27,3 |
| E7 | 82,9 | -1,4 | 12,1 | 57,6 | -2,0 | 4,4 | 26,5 |
| E8 | 83,2 | -1,5 | 11,6 | 59,5 | -2,1 | 3,5 | 25,0 |
| E9 | 83,2 | -1,6 | 11,0 | 61,7 | -2,1 | 3,0 | 23,0 |
| E10 | 85,7 | 0,0 | 9,8 | 47,4 | -1,6 | 4,0 | 38,8 |
| E11 | 85,7 | -0,3 | 10,9 | 50,0 | -2,0 | 3,7 | 36,4 |
| E12 | 83,6 | -0,5 | 12,3 | 54,2 | -2,3 | 3,0 | 30,9 |
| E13 | 84,8 | -0,5 | 10,9 | 57,0 | -2,3 | 1,8 | 29,3 |
| E14 | 86,5 | -0,8 | 10,1 | 56,2 | -2,6 | 1,2 | 31,6 |
| C1 | 84,8 | -0,9 | 9,8 | 54,9 | -2,6 | 1,0 | 31,2 |

Light scattering measurements

The compositions according to Example 3 (E3), Comparative example C1 (Venus Diamond One) and Comparative example C3 (Tetric Prime) were evaluated in this study. Test specimens were shaped with a mould (diameter 15 mm and height 1 mm), light-cured by G-light Prima II Plus (GC corp.) from nine spots from both sides and both sides were also polished with #1000 silicon carbide (SiC) paper. The intensity of transmitted light at various angles (-90° to +90°) were measured by goniophotometer (Murakami Color Research Lab.).

The specimen was attached on to a lift table with a piece of Blue-Tack. A pointer pen was set in to certain distance from the specimen. The pointers used were coloured green, red and blue. The pointer's laser was pointed through the specimen on to a piece of white copy paper. On to the paper was printed a series of circles to analyse the light's scatter with a visual inspection.

Further, goniophotometer measurements were made for the same samples, and its results are shown in Figure 2. Figure 2 also contains goniophotometer measurement results for the Comparative examples C1 and C3.

Both light scattering measurements showed that the dental composite according to the present invention showed a dentin-like light scattering effect while the commercial products had an enamel-like effect.

Evaluation of chameleon effect

Photos of the tooth models with restorations as prepared in Example 2 and Comparative examples C1 and C4 are shown in the Figures.

Figure 3 shows the first caries in the first dental model. Figure 4 shows the four different shades (A1, A3, A4 and C3) and the restorations, the uppermost line for Omnicroma (Comparative example C4), the middle line for Venus Diamond One (Comparative example C1) and the lowest line for the composition of Example 2. Figure 5 is the same as Figure 4, but in black and white.

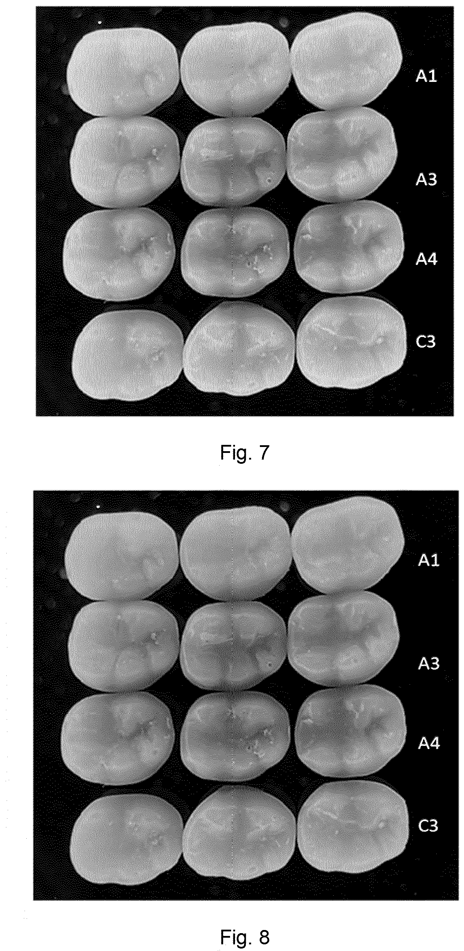

Figure 6 shows the second caries in the second dental model. Figure 7 shows the four different shades (A1, A3, A4 and C3) and the restorations, the left-most column for Venus Diamond One (Comparative example C1) the middle column for Omnicroma (Comparative example C4), and the right-most column for the composition of Example 2. Figure 8 is the same as Figure 7, but in black and white.

The photos show that all the restorations prepared according to the present Examples had the same chameleon effect and the commercial restorations.

Flexural strength and modulus

Flexural strength and modulus were measured according to ISO 4049:2019. Three-point bending test specimens ($2 \times 2 \times 25$ mm$^3$) were made from each tested composite. Bar-shaped specimens were made in half-split stainless-steel moulds between transparent Mylar sheets (100 $\mu$m). Polymerisation of the materials was done using a hand light-curing unit (Elipar S10, 3M ESPE, St. Paul, MN, USA) for 20 s in five separate overlapping portions from both sides of the metal mould. The wavelength of the light was between 430 and 480 nm and light intensity was 1600 mW/cm$^2$. The specimens from each material (n=8) were stored in dry atmosphere at 37 °C for one day before testing. The three-point bending test was conducted according to the ISO 4049:2009 (test span: 20 mm, cross-head speed: 1 mm/min, indenter: 2 mm diameter). All specimens were loaded into a material testing machine (model LR30K+, Lloyd Instrument Ltd., Fareham, England) and the load-deflection curves were recorded with PC-computer software (Nexygen 4.0, Lloyd Instruments Ltd., Fareham, England).

Results as Young's modulus of bending (GPa) and maximum bending stress at maximum load (MPa) are given in Table 18. All samples were dry.

Table 18

| Example | Young's modulus of bending (GPa) | Maximum bending stress at maximum load (MPa) |
|---------|----------------------------------|----------------------------------------------|
| E5 | 11.5 | 156 |
| E6 | 12.1 | 175 |
| E7 | 12.6 | 183 |
| E8 | 12.9 | 194 |
| E9 | 12.9 | 185 |
| E10 | 9.6 | 140 |
| E11 | 9.6 | 149 |
| E12 | 9.8 | 145 |
| E13 | 9.5 | 151 |
| E14 | 9.8 | 146 |
| C1 | 14.8 | 198 |

## Claims

1. A dental restorative composition, comprising 10-30 wt-% of a resin and 45-80 wt-% of silanated inert glass particles having an average particle size of 0.2-0.9 $\mu$m.

2. The dental restorative composition according to claim 1, wherein the amount of the silanated inert glass particles is 45-60 wt-%, and the composition further comprises 20-30 wt-% of inert glass fibres, the glass fibres having a diameter of 4-8 $\mu$m and a distribution of length of 50-200 $\mu$m.

3. The dental restorative composition according to any of the preceding claims, wherein the amount of resin is 15-25 wt-%.

4. The dental restorative composition according to any of the preceding claims, wherein the average particle size of the silanated inert glass particles is 0.4-0.7 $\mu$m.

5. The dental restorative composition according to any of the preceding claims, wherein the inert glass particles are selected from E-glass particles, S-glass particles and mixtures thereof.

6. The dental restorative composition according to any of the claims 2-4, wherein the inert glass fibres are selected from E-glass fibres, S-glass fibres and mixtures thereof.

7. The dental restorative composition according to any of the claims 2-6, wherein the amount of glass fibres is 24-28 wt-%.

8. The dental restorative composition according to any of the preceding claims, wherein the resin is selected from a group consisting of methyl methacrylate, ethyl methacrylate, n-butyl methacrylate, isobutyl methacrylate, 2-ethylhexyl methacrylate, cyclohexyl methacrylate, isobornyl methacrylate, tetrahydrofurfuryl methacrylate, benzyl methacrylate, morpholinoethyl methacrylate, diethylene glycol dimethacrylate, triethylene glycol dimethacrylate, diurethane dimethacrylate, 2,2-bis(4-(2-hydroxy-3-methacryloxy)phenyl)propane, acrylic acid, epoxy, bisphenol A-glycidyl methacrylate (bis-GMA), bisphenol A ethoxylate dimethacrylate (bis-EMA), urethane dimethacrylate (UDMA), trimethylolpropane ethoxylate triacrylate and mixtures thereof.

9. The dental restorative composition according to any of the preceding claims, wherein the degree of silanation of the inert glass particles is 1-15 wt-%.

Fig. 1

Overlay

E3

C1

C3

Fig. 2

Fig. 3

Fig. 4

Fig. 5

Fig. 6

Fig. 7

Fig. 8

## EUROPEAN SEARCH REPORT

Europäisches
Patentamt
European
Patent Office
Office européen
des brevets

**Application Number**

EP 24 20 6569

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2023/059534 A1 (BUSCH SUSANNE [AT] ET AL) 23 February 2023 (2023-02-23) | 1,3-5,8,9 | INV.<br>A61K6/17 |
| A | * paragraphs [0001], [0178], [0182], [0185], [0189], [0194] *<br>* example 1 *<br>* table 1 *<br>----- | 2,6,7 | A61K6/77<br>A61K6/887 |
| X | BEHL SONAM ET AL: "Physical and mechanical characterisation of flowable dental composites reinforced with short aspect ratio micro-sized S-Glass fibres", MATERIALS SCIENCE AND ENGINEERING C, ELSEVIER SCIENCE S.A, CH, vol. 111, 28 February 2020 (2020-02-28), XP086129844, ISSN: 0928-4931, DOI: 10.1016/J.MSEC.2020.110771 [retrieved on 2020-02-28]<br>* sections 1, 2.1 and 4 *<br>* table 1 *<br>----- | 1-9 | |

**TECHNICAL FIELDS SEARCHED (IPC)**

A61K

The present search report has been drawn up for all claims

| Place of search | Date of completion of the search | Examiner |
|---|---|---|
| The Hague | 29 April 2025 | Grave, Christian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 20 6569

This annex lists the patent family members relating to the patent documents cited in the above-mentioned European search report.
The members are as contained in the European Patent Office EDP file on
The European Patent Office is in no way liable for these particulars which are merely given for the purpose of information.

29-04-2025

| Patent document cited in search report | Publication date | Patent family member(s) | | Publication date |
|---|---|---|---|---|
| US 2023059534 A1 | 23-02-2023 | CN | 114845683 A | 02-08-2022 |
| | | EP | 3854374 A1 | 28-07-2021 |
| | | JP | 2023510962 A | 15-03-2023 |
| | | US | 2023059534 A1 | 23-02-2023 |
| | | WO | 2021148667 A1 | 29-07-2021 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **LASSILA et al.** Mechanical properties of fiber reinforced restorative composite with two distinguished fiber length distribution. *Journal of the mechanical behavior of biomedical materials*, 2016, vol. 60, 331-338